(19) Europäisches Patentamt
European Patent Office
Office européen des brevets

(11) Veröffentlichungsnummer: **0 055 354**
**B1**

(12) **EUROPÄISCHE PATENTSCHRIFT**

(45) Veröffentlichungstag der Patentschrift: **12.09.84**

(51) Int. Cl.³: **C 07 C 45/38**, C 07 C 45/81, C 07 C 47/21

(21) Anmeldenummer: **81108836.8**

(22) Anmeldetag: **24.10.81**

(54) **Verfahren zur kontinuierlichen Herstellung von 3-Alkyl-buten-1-alen.**

(30) Priorität: **31.12.80 DE 3049543**

(43) Veröffentlichungstag der Anmeldung: **07.07.82 Patentblatt 82/27**

(45) Bekanntmachung des Hinweises auf die Patenterteilung: **12.09.84 Patentblatt 84/37**

(84) Benannte Vertragsstaaten: **BE CH DE FR GB IT LI NL**

(56) *Entgegenhaltungen:*
**EP-A-0 008 013**
**EP-A-0 019 772**
**FR-A-2 386 509**

(73) Patentinhaber: **BASF Aktiengesellschaft, Carl-Bosch-Strasse 38, D-6700 Ludwigshafen (DE)**

(72) Erfinder: **Sauer, Wolfgang, Dr., Langstrasse 23, D-6719 Kirchheimbolanden (DE)**
Erfinder: **Aquila, Werner, Dr., Meissener Weg 35, D-6800 Mannheim (DE)**
Erfinder: **Hoffmann, Wolfgang, Dr., Albert-Haueisen-Strasse 4, D-6710 Frankenthal (DE)**
Erfinder: **Brenner, Karl, Riedsaumstrasse 40, D-6700 Ludwigshafen (DE)**
Erfinder: **Halbritter, Klaus, Dr., Schwarzwaldstrasse 19, D-6800 Mannheim (DE)**

EP 0 055 354 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur kontinuierlichen Herstellung von 3-Alkyl-buten-1-alen durch oxidierende Dehydrierung von 3-Alkyl-buten-1-olen an Katalysatoren aus Kupfer und/oder Silber.

Aus der DE-OS Nr. 2715209 ist bekannt, dass man 3-Alkyl-buten-1-ale nach einem kontinuierlichen Verfahren durch oxydierende Dehydrierung der entsprechenden Alkohole mit Sauerstoff an einer Katalysatorschicht aus Silber und/oder Kupfer herstellen kann. Die Umsetzung wird bei Temperaturen von 320 bis 650° C durchgeführt. Die heissen Reaktionsgase werden abgekühlt und die 3-Alkyl-buten-1-ale abgetrennt. Aus dem bevorzugten Ausgangsstoff 3-Methyl-3-buten-1-ol erhält man bei Umsätzen von 80,7 bzw. 84,1% Ausbeuten an 3-Methyl-buten-1-al von 76,6 bzw. 81%, bezogen auf umgesetztes 3-Methyl-3-buten-1-ol.

Dieses Verfahren ist in bezug auf einen einfachen und wirtschaftlichen Betrieb, eine gute Ausbeute und Reinheit der Aldehyde sowie die Abtrennung der Nebenstoffe unbefriedigend. Auch ist es nachteilig, dass häufig die Abkühlungsanlagen innerhalb kurzer Zeit verstopfen, so dass ein kontinuierlicher Betrieb beeinträchtigt wird. Die sich zum Teil in den anfallenden Reaktionsprodukten lösenden Crackprodukte und Polymeren verunreinigen die Produktlösungen so, dass bei der Weiterverarbeitung Probleme auftreten, wie vermehrte Bildung von Rückständen und Ausbeuteverluste bei weiteren Umsetzungen des Rohproduktes. Ausserdem ist ein hoher Aufwand bei der Reinigung des Rohprodukts notwendig, um die Nebenprodukte zu entfernen.

In der EP-A Nr. 19772 wird ein Verfahren zur kontinuierlichen Herstellung von Glyoxal durch Oxidation von Glykol beschrieben, bei dem man aus dem heissen Strom des dampfförmigen Reaktionsgemisches nach der Oxidation und höchstens eine Sekunde nach Austritt aus dem Silberkatalysator mittels Wasser oder Produktlösung in Form von Tröpfchen von einem durchschnittlichen Durchmesser von 1 bis 2000 Mikrometern das Produktgemisch kondensiert, wobei die Hauptmenge der Tröpfchen auf die Reaktionsgase mit einem Winkel von 2 bis 85° C zur Stromachse auftrifft.

Es wurde nun gefunden, dass man bei der kontinuierlichen herstellung von 3-Alkyl-buten-1-alen der Formel

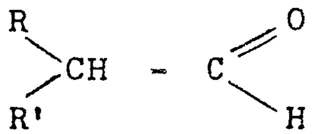 I

in der R ein Wasserstoffatom und R' einen $H_2C=C(CH_3)$-Rest oder beide Reste R und R' zusammen den Rest der Formel $H_3C-C(CH_3)=$ bedeuten, durch oxidierende Dehydrierung von 3-Alkyl-buten-1-olen der Formel

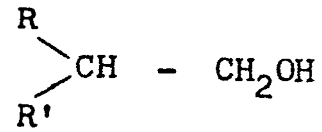 II

in der R und R' die obengenannte Bedeutung haben, bei der man die 3-Alkyl-buten-1-ole bei Temperaturen von 320 bis 650° C mit Sauerstoff über einen Kupfer und/oder Silber enthaltenden Katalysator leitet, die Reaktionsgase abkühlt und die 3-Alkyl-buten-1-ale abtrennt, erheblich vorteilhaftere Ergebnisse erzielt, wenn man das dampfförmige Reaktionsgemisch, das eine Temperatur von 320 bis 650° C aufweist, innerhalb von einer Sekunde nach der Berührung mit dem Katalysator mit einer Flüssigkeit aus Wasser und/oder dem kondensierten Reaktionsgemisch der Temperatur von −20 bis 50° C in Berührung bringt und aus dem erhaltenen Kondensat die 3-Alkyl-buten-1-ale abtrennt.

Im Vergleich zu den bekannten Verfahren liefert das Verfahren nach der Erfindung überraschend auf einfacherem und wirtschaftlicherem Wege ein besseres Gesamtergebnis mit Bezug auf Ausbeute und Reinheit der Endstoffe.

Die oxidierende Dehydrierung der 3-Alkyl-buten-1-ole mit Sauerstoff an dem Katalysator führt man zunächst auf an sich bekannte Weise, z.B. wie in der DE-OS Nr. 2715209 beschrieben, bei Temperaturen von 320 bis 650° C, vorzugsweise 400 bis 600° C durch. Das dabei erhältliche dampfförmige Reaktionsgemisch, das eine Temperatur von 320 bis 650° C aufweist, wird dann nach dem erfindungsgemässen Verfahren unmittelbar nach dem Verlassen der Katalysatorschicht mit einer Flüssigkeit in Berührung gebracht, die die unverzügliche Kondensation der Oxidationsprodukte bewirkt. Als Kondensationsflüssigkeit verwendet man Wasser, das kondensierte flüssige Reaktionsgemisch oder ein Gemisch dieser beiden Flüssigkeiten. Bevorzugt verwendet man die wässerige oder die organische Phase des bei der Kondensation der Reaktionsgemische anfallenden zweiphasigen Kondensates. Nach einer besonders vorteilhaften Ausführungsform der Erfindung wird die wässerige Phase als Kondensationsflüssigkeit verwendet. Bei der Verwendung von kondensierten Reaktionsgemischen als Kondensationsflüssigkeit wird der überschüssige Teil an Kondensat, welcher nicht als gekühlte Kondensationsflüssigkeit im Kreis geführt wird, als Rohprodukt entnommen und gegebenenfalls einer Aufarbeitung oder weiteren Umsetzung zugeführt.

Das bei der Oxidation erhältliche dampfförmige Reaktionsgemisch, das, wie beschrieben, zur Kondensation der Verfahrensprodukte mit der Kondensationsflüssigkeit behandelt wird, hat z.B. folgende Zusammensetzung:

34,5% 3-Methyl-3-buten-1-al, 6,7% 3-Methyl-2-buten-1-al, 12,6% 3-Methyl-3-buten-1-ol, 1,2% hochsiedende organische Produkte, 0,3% Säuren, 0,3% Formaldehyd, 2,1% Isobuten, 2,2% Kohlenmonoxid, 2,4% Kohlendioxid, 13,4% Wasser, 0,7% Wasserstoff, 23,6% Stickstoff.

Wesentlich ist, dass man das dampfförmige Reaktionsgemisch, das eine Temperatur von 320 bis 650° C aufweist, innerhalb von einer Sekunde mit der Kondensationsflüssigkeit in Berührung bringt. Die Temperatur der Kondensationsflüssigkeit beträgt dabei vorzugsweise −20 bis 50° C. Man führt diese Behandlung z.B. so durch, dass man den Strom der heissen Reaktionsgase direkt in einen Quenchraum leitet, der sich in Strömungsrichtung unmittelbar an die Katalysatorschicht anschliesst. Man verfährt dabei vorzugsweise so, dass man die Kondensationsflüssigkeit in Form von Tröpfchen mit einem durchschnittlichen Durchmesser von 1 bis 2000 Mikrometern mit den Reaktionsgasen in Berührung bringt. Die Tröpfchen werden mit Hilfe von an sich üblichen Zerteilungsvorrichtungen, insbesondere von Düsen, erzeugt. Das Einsprühen der Kondensationsflüssigkeit in den Quenchraum nimmt man vorteilhaft so vor, dass die Hauptmenge der Tröpfchen auf den Strom der Reaktionsgase mit einem Winkel von 2 bis 85° zur Stomachse auftrifft.

Die Menge an Kondensationsflüssigkeit (in den Beispielen auch als Quenchflüssigkeit bezeichnet) beträgt zweckmässigerweise 20 bis 100 Gewichtsteile, bezogen auf 1 Gewichtsteil der Reaktionsgase. Nach erfolgter Kondensation hat das flüssige Kondensat z.B. folgende Zusammensetzung:50,1% 3-Methyl-3-buten-1-al, 9,6% 3-Methyl-2-buten-1-al, 18,3% 3-Methyl-3-buten-1-ol, 1,7% hochsiedende organische Produkte, 0,5% Säuren, 0,5% Formaldehyd, 19,3% Wasser.

Die Aufarbeitung der Kondensate zur Abtrennung der 3-Alkyl-buten-1-ale wird auf an sich üblichem Wege so durchgeführt, indem man das Kondensat z.B. einer fraktionierten Destillation unterzieht. Hierbei können die hergestellten 3-Alkyl-buten-1-ale als leichtersiedende Komponenten von dem Ausgangsmaterial, 3-Alkyl-buten-1-ol agbetrennt und isoliert werden. Ist die Herstellung des 3-Alkyl-2-buten-1-als erwünscht, so kann das nach dem erfindungsgemässen Verfahren hergestellte 3-Alkyl-3-buten-1-al nach der in der DE-OS Nr. 2715208 und der DE-OS Nr. 2715209 beschriebenen Weise katalytisch direkt im Reaktionsgemisch isomerisiert und anschliessend durch fraktionierte Destillation isoliert werden.

Durch das erfindungsgemässe Verfahren werden die Ausbeuten bei der Umsetzung von 3-Methyl-3-buten-1-ol um etwa 10% gesteigert. Gleichzeitig wird der Gehalt an luftfremden Stoffen im Abgas wie Kohlenmonoxid, Kohlenwasserstoffe und Formaldehyd, zum Teil deutlich verringert, so dass die Reinigung der Abgase verbessert werden kann. Ferner wird die Aufarbeitung der Rohprodukte wegen des geringeren Nebenproduktspiegels, insbesondere an Hochsiedern und hochsiederbildenden Komponenten, vereinfacht. Im Vergleich zum Verfahren der DE-OS Nr. 2715209 wird eine Zersetzung der heissen Reaktionsgase und Verstopfungen der Rohrverbindungen und Anlagen bei der Abkühlung vermieden oder entscheidend verringert. Damit wird der störungsfreie Betrieb der Anlagen deutlich verbessert und die Wirtschaftlichkeit erhöht.

Weitere Vorteile des erfindungsgemässen Verfahrens ergeben sich auch im Hinblick auf eine direkte Weiterverarbeitung der Rohprodukte ohne vorherige Aufarbeitung, da die erhaltenen Rohprodukte hinsichtlich ihrer Reinheit, z.B. nach Farbzahl, Säurezahl und Rückstandsgehalt, im Vergleich besser sind, so dass auch die Folgeprodukte in besserer Ausbeute und Reinheit hergestellt werden können.

Diese vorteilhaften Ergebnisse waren nicht zu erwarten, da die 3-Alkyl-buten-1-ale als ungesättigte aliphatische Aldehyde bekanntlich sehr reaktionsfähige Stoffe darstellen (s. Ullmann, 4. Auflage, Bd. 7, Seite 118 ff., insbes. 130-133). Sie werden z.B. leicht zu den entsprechenden Carbonsäuren oxidiert, bilden Diels-Alder-Addukte, polymerisieren leicht und sind Partner für eine Vielzahl von Additionsreaktionen. Viele dieser Reaktionen werden durch ein polyres Medium und Spuren von Säuren, Basen oder Salzen beschleunigt.

Es ist daher überraschend, dass es beim Kontaktieren der heissen Reaktionsgase mit der feinverteilten Kondensationsflüssigkeit nicht zu vermehrter Nebenproduktbildung kommt, zumal dem 3-Alkyl-buten-1-al, verglichen mit dem Verfahren der DE-AS Nr. 2715209, eine deutlich grössere Kontaktfläche zu den im Reaktionsgas enthaltenen, nicht kondensierenden reaktiven Komponenten, wie Sauerstoff, Wasserstoff, Kohlenmonoxid, Formaldehyd, Isobuten, angeboten wird und ausserdem noch Wasser und andere, die Nebenproduktbildung fördernde Begleitstoffe, wie Säuren, zugegeben sind. Besonders bei der bevorzugten Ausführungsform mit im Kreis geführter wässeriger Produktlösung als Kondensationsflüssigkeit war ein Ansteigen der Nebenproduktbildung zu erwarten, da der sehr reaktive ungesättigte Aldehyd immer wieder in der für eine Reaktion sehr förderlichen feinverteilten Tröpfchenform den heissen Reaktionsgasen ausgesetzt wird.

Da Glyoxal im Vergleich zu den 3-Alkyl-buten-1-alen chemisch erheblich weniger empfindlich ist, hat es nicht nahegelegen, die aus der DE-OS Nr. 2715209 bekannte Oxidation vom 3-Alkyl-buten-1-olen zu den entsprechenden Aldehyden unter Verwendung der in der EP-A Nr. 19772 beschriebenen Verfahrensmassnahmen durchzuführen.

Die in den folgenden Beispielen angeführten Teile bedeuten Gewichtsteile.

*Beispiel 1* (Figur 1)

Man verwendet eine Anlage mit Alkohol-Verdampfer (1), entsprechenden Zuführungen von Alkohol (6), Wasser (7) und Luft bzw. Stickstoff (11) und einen senkrechten Rohrreaktor (2). Der Reaktor enthält an seinem Kopf die Zuführung (12) für das dampfförmige Ausgangsgemisch und die Reaktorhaube. Die Katalysatorschicht liegt unterhalb des Reaktorkopfes, unmittelbar daran schliesst sich der Quenchraum (10) an, der den Kopf einer Füllkörperkolonne (3) bildet. Zur Abnahme des kondensierten flüssigen Reaktionsproduktes befindet sich am unteren Ende der Kolonne eine Leitung (8). Um die flüssigen Reaktionsprodukte teilweise als Quenchflüssigkeit verwenden zu können, ist die Leitung (8) mit einer Pumpe (4) verbunden, durch die die Flüssigkeit über einen Wärmetauscher (5) und ein Düsensystem in den Quenchraum gesprüht werden kann. Das Abgas entweicht über die Leitung (9).

In den Reaktor (2) wird ein Katalysator aus Silberkristallen (28 Teilen) folgender Zusammensetzung eingetragen:

| | Anteil am Katalysator Gewichtsprozent | Korngrösse mm |
|---|---|---|
| Schicht 1 (oben) | 21,4 | 0,2 bis 0,4 |
| Schicht 2 (Mitte) | 50,0 | 0,4 bis 0,75 |
| Schicht 3 (Mitte) | 23,6 | 0,75 bis 1,0 |
| Schicht 4 (unten) | 5,0 | 1,0 bis 2,5 |

Die Gesamtschichtdicke des Katalysators vor Beginn der Reaktion beträgt 20 mm.

Der Katalysator wird von aussen auf eine Temperatur von 460° C erhitzt. In dem Verdampfer (1) werden über Leitung (6) 80 Teile 3-Methyl-3-buten-1-ol eingegeben und auf 125° C erwärmt. Nun werden über Leitung (11) in den Verdampfer 100 Teile Luft pro Stunde eingeleitet. Das gasförmige Ausgangsgemisch strömt durch die Leitung (12) in den Reaktor (10), wobei die Temperatur des Katalysators zu steigen beginnt. Mit Beginn der Reaktion, erkenntlich durch die steigende Katalysatortemperatur, werden innerhalb von 0,1 Stunden die Luftmenge auf stündlich 182 Teile Luft und gleichzeitig die durch den Katalysator geleitete Menge an 3-Methyl-3-buten-1-ol auf 361 Teile pro Stunde gebracht. Dies entspricht einer Katalysatorbelastung von 1,15 t 3-Methyl-3-buten-1-ol pro Stunde und Quadratmeter Katalysatorbettquerschnitt. Zusätzlich werden pro Stunde 64 Teile Wasser über Leitung (7), Verdampfer (1) und Leitung (12) gasförmig auf den Katalysator geleitet. Nach Wegnahme der Aussenheizung stellt sich eine Reaktionstemperatur im Silber von 485° C und ein Druck vor dem Katalysator von 1,10 bar ein. Die Verweilzeit der Gase im Katalysatorraum beträgt 0,008 Sekunden.

Die die Katalysatorschicht mit 485° C verlassenden Reaktionsgase werden im Quenchraum (10) mit der Quenchflüssigkeit in Form von Tröpfchen kontaktiert. Zur Erzeugung der Tröpfchen dienen 2 Ringe mit jeweils 6 auf Lücke angeordneten Düsen, die an der Wand der Kolonne (3) symmetrisch angeordnet sind. Die Düsen werden über die Pumpe (4) und den Wärmetauscher (5) mit dem heterogenen Gemisch der bei der Reaktion anfallenden wässerigen und organischen Produktphase, welche man aus der Ableitung (8) abzweigt, als Quenchflüssigkeit im Kreislauf beschickt. Der Auftreffwinkel der Tröpfchen zur Stromachse aller Düsen ist unterschiedlich und beträgt 15 bis 75°. Bei 70% der Tröpfchen beträgt dieser Winkel 30 bis 75°. Die Tröpfchen haben einen durchschnittlichen Durchmesser von 200 Mikrometern.

Die Verweilzeit der Reaktionsgase zwischen Katalysatorschicht und Quenchraum beträgt 0,05 Sekunden, die Verweilzeit im Quenchraum 1 Sekunde. Die Temperatur der Gase vor Eintritt in den Quenchraum beträgt 470° C, die der Quenchflüssigkeit 1° C, die Temperatur der Gase nach dem Austritt aus dem Quenchraum 16° C, die Menge der Quenchflüssigkeit 30 000 Teile pro Stunde.

Über die Leitung (8) wird kontinuierlich die bei der Reaktion pro Zeiteinheit anfallende Menge an heterogenem Gemisch der wässerigen und organischen Phase abgezogen.

Pro Stunde fallen 203 Teile 3-Methyl-3-buten-1-al sowie 39 Teile 3-Methyl-2-buten-1-al neben 74 Teilen unumgesetztem 3-Methyl-3-buten-1-ol an. Der Umsatz beträgt 79,5%, die Gesamtausbeute an 3-Methylbutenal 86,3% der Theorie, bezogen auf umgesetztes 3-Methyl-buten-1-ol.

In der entnommenen Produktlösung fallen pro Stunde 7 Teile hochsiedende Kondensations- und Polymerisationsprodukte als unerwünschte Verunreinigung an. Als weitere wesentliche Nebenprodukte werden über die Leitung (9) im Abgas der Reaktion 12 Teile Isobutylen, 13 Teile Kohlenmonoxid und 14 Teile Kohlendioxid abgeführt.

Die Synthese wurde 60 Tage in der geschilderten Weise störungsfrei betrieben, ohne dass es zu Verstopfungen infolge von Ablagerungen im Quenchraum oder in der nachgeschalteten Füllkörperkolonne wäre. Ausbeute und Produktqualität blieben dabei unverändert.

*Beispiel 2* (Figur 2)

In einer Anlage nach Figur 2, die zusätzlich zu der Anlage gemäss Figur 1 einen Phasenscheider (14) enthält, wird analog Beispiel 1 pro Stunde ein Gemisch aus 361 Teilen 3-Methyl-3-buten-1-ol und 64 Teilen Wasser mit 182 Teilen Luft bei 485° C und einem Druck von 1,10 bar über die Katalysatorschüttung geführt. Die Verweilzeit der Gase im Katalysatorraum beträgt 0,008 Sekunden. Die den Katalysatorraum (2) verlassenden Reaktionsgase werden in dem Quenchraum (10) mit einer zu Tröpfchen verdüsten Quenchflüssigkeit kontaktiert, die aus der wässerigen Phase (16) des in dem Phasentrenngefäss (14) in die wässerige und organische Phase getrennten Reaktionsaustrags besteht. Die Quenchflüssigkeit wird über die Pumpe (4) und den Wärmetauscher (5) im Kreis geführt. Die pro Zeiteinheit anfallende Menge an wässeriger Phase wird kontinuierlich über die Leitung (8) aus dem Kreislauf ausgeschleust. Die anfallende organische Phase (15) wird über die Leitung (17) abgezogen.

Die im Quenchraum erzeugten Tröpfchen haben einen durchschnittlichen Durchmesser von 200 Mikrometern. Ihr Auftreffwinkel zur Stromachse beträgt 15 bis 75°. Bei 70% der Tröpfchen beträgt dieser Winkel 30 bis 75°.

Die Verweilzeit der Reaktionsgase zwischen Katalysator und Quenchraum beträgt 0,05 Sekunden, die Verweilzeit im Quenchraum 1 Sekunde.

Die Temperatur der Gase vor Eintritt in den Quenchraum beträgt 470° C, die der Quenchflüssigkeit 1° C, die Temperatur der Gase nach dem Austritt aus dem Quenchraum 11° C, die Menge der Quenchflüssigkeit 30 000 Teile pro Stunde.

Über die Leitungen (8) und (17) werden pro

Stunde 199 Teile 3-Methyl-3-buten-1-al sowie 45 Teile 3-Methyl-2-buten-1-al neben 74 Teilen unumgesetztem 3-Methyl-3-buten-1-ol entnommen. Der Umsatz beträgt 79,5%, die Gesamtausbeute an 3-Methylbutenal 87,0%, bezogen auf umgesetztes 3-Methyl-3-buten-1-ol.

In der entnommenen Produktlösung fallen pro Stunde 7 Teile hochsiedende Kondensations- und Polymerisationsprodukte als unerwünschte Verunreinigung an. Als weitere wesentliche Nebenprodukte werden über die Leitung (9) im Abgas der Reaktion 11 Teile Isobutylen, 12 Teile Kohlenmonoxid und 14 Teile Kohlendioxid abgeführt.

Die Synthese wurde 60 Tage in dieser Form störungsfrei betrieben, ohne dass es zu Verstopfungen infolge von Ablagerungen im Quenchraum oder in der nachgeschalteten Füllkörperkolonne gekommen wäre. Ausbeute und Produktqualität blieben dabei unverändert.

*Beispiel 3* (Figur 3)

In einer Variante der Anlage, die in der Figur 3 dargestellt ist, wird analog Beispiel 1 pro Stunde ein Gemisch aus 361 Teilen 3-Methyl-3-buten-1-ol und 64 Teilen Wasser mit 182 Teilen Luft bei 485° C und einem Druck von 1,10 bar über die Katalysatorschüttung geführt. Die Verweilzeit der Gase im Katalysatorraum beträgt 0,008 Sekunden.

Die den Katalysatorraum (2) verlassenden Reaktionsgase werden in den Quenchraum (10) mit einer zu Tröpfchen verdüsten Quenchflüssigkeit kontaktiert, die aus der organischen Phase (15) des in dem Phasentrenngefäss (14) in die wässerige und organische Phase getrennten Reaktionsaustrags besteht. Die Quenchflüssigkeit wird über die Pumpe (4) und den Wärmetauscher (5) im Kreis geführt. Die pro Zeiteinheit anfallende Menge an organischer Phase wird kontinuierlich über die Leitung (8) aus dem Kreislauf ausgeschleust. Die anfallende wässerige Phase wird über die Leitung (18) abgezogen.

Die im Quenchraum erzeugten Tröpfchen haben einen durchschnittlichen Durchmesser von 200 Mikrometern. Ihr Auftreffwinkel zur Stromachse beträgt 15 bis 75°. Bei 70% der Tröpfchen beträgt dieser Winkel 30 bis 75°.

Die Verweilzeit der Reaktionsgase zwischen Katalysator und Quenchraum beträgt 0,05 Sekunden, die Verweilzeit im Quenchraum 1 Sekunde. Die Temperatur der Gase vor Eintritt in den Quenchraum beträgt 470° C, die der Quenchflüssigkeit −10° C, die Temperatur der Gase nach dem Austritt aus dem Quenchraum 9° C, die Menge der Quenchflüssigkeit 30 000 Teile pro Stunde.

Über die Leitungen (8) und (18) werden pro Stunde 205 Teile 3-Methyl-3-buten-1-al sowie 37 Teile 3-Methyl-2-buten-1-al neben 74 Teilen unumgesetztem 3-Methyl-3-buten-1-ol entnommen. Der Umsatz beträgt 79,5%, die Gesamtausbeute an 3-Methylbutenal 86,3%, bezogen auf umgesetztes 3-Methyl-3-buten-1-ol.

In der entnommenen Produktlösung fallen pro Stunde 8 Teile hochsiedende Kondensations- und Polymerisationsprodukte als unerwünschte Verunreinigung an. Als weitere wesentliche Nebenprodukte werden über die Leitung (9) im Abgas der Reaktion 12 Teile Isobutylen, 12 Teile Kohlenmonoxid und 14 Teile Kohlendioxid abgeführt.

Die Synthese wurde 60 Tage in dieser Form störungsfrei betrieben, ohne dass es zu Verstopfungen infolge von Ablagerungen im Quenchraum oder in der nachgeschalteten Füllkörperkolonne gekommen wäre. Ausbeute und Produktqualität blieben dabei unverändert.

*Vergleichsbeispiel* (Figur 4)

Man verwendet eine Anlage, die gemäss Figur 4 aus einem Verdampfer (1), einem senkrechten Rohrreaktor (19), einem nachgeschalteten Kühler (20) und einer Absorptionsanlage (21) besteht. Der Verdampfer ist durch die Leitung (28) mit dem Rohrreaktor verbunden. Die Leitung ist bis zum Reaktor hin beheizbar. Die Katalysatorschicht liegt unterhalb des Reaktorkopfes, weiter unten folgt die Kühlzone (20). Das dort in der Kühlzone gebildete Kondensat sammelt sich in der Vorlage (22), die Kühlzone ist über eine Pumpe (4) und die Leitung (23) mit der Absorptionsanlage (21) verbunden. Den Absorptionsteil bilden zwei in Kaskade angeordnete doppelwandige Absorptionskolonnen mit Aussenmantelkühlung. Die beiden Kolonnen sind mit 10-mm-Raschigringen aus Glas gefüllt. Über die Leitung (24) entweicht das Abgas.

In den Reaktor (19) wird, wie in Beispiel 1 beschrieben, ein Katalysator eingefüllt. Analog Beispiel 1 werden durch die Zuleitungen (6), (7) und (11) pro Stunde 361 Teile 3-Methyl-3-buten-1-ol, 64 Teile Wasser und 182 Teile Luft bei 485° C und einem Druck von 1,08 bar über die Katalysatorschicht geführt. Die Verweilzeit der Gase im Katalysatorraum beträgt 0,008 Sekunden.

Das Reaktionsgemisch wird anschliessend in der Kühlzone des Reaktors (20) auf 25° C abgekühlt, wobei ein Teil kondensiert und in einer Vorlage (22) gesammelt wird. Die Absorption in (21) erfolgt in zwei Stufen im Gegenstrom in Form einer Gaswäsche. Als Absorptionsflüssigkeit werden über Leitung (25) pro Stunde 840 Teile Dimethylformamid mit einer Temperatur in der Kaskade (21) von −10° C eingeleitet. Das im Kondensationsteil und Absorptionsteil über die Leitungen (26) und (27) erhaltene Reaktionsgemisch wird destilliert, wobei wegen der katalytischen Eigenschaft des als Absorptionsflüssigkeit verwendeten Dimethylformamids das im Reaktionsgemisch vorhandene 3-Methyl-3-buten-1-al quantitativ zum 3-Methyl-2-buten-1-al isomerisiert.

Man erhält pro Stunde 218 Teile 3-Methyl-2-buten-1-al neben 70 Teilen unumgesetztem 3-Methyl-3-buten-1-ol. Der Umsatz beträgt 80,6%, die Gesamtausbeute an 3-Methylbutenal 76,7%, bezogen auf umgesetztes 3-Methyl-3-buten-1-ol.

In der entnommenen Produktlösung fallen pro Stunde 13 Teile hochsiedende Kondensations- und Polymerisationsprodukte als unerwünschte Verunreinigung an. Als weitere wesentliche Nebenprodukte werden über die Leitung (24) im Abgas der Reaktion 23 Teile Isobutylen, 23 Teile

Kohlenmonoxid und 14 Teile Kohlendioxid abgeführt.

Im Laufe einer Betriebsdauer von 14 Tagen stieg der Druckverlust des Systems von dem Anfangswert von 1,08 bar auf 1,95 bar an. Ursache hierfür war eine immer mehr zunehmende Verstopfung des Kühlers (20) durch sich ablagernde Crackprodukte. Parallel dazu stieg der Hochsiederanfall im kondensierten Produkt von 13 Teilen auf 19 Teile pro Stunde an bei gleichzeitiger deutlicher Zunahme der Verfährbung des Kondensats durch gelöste Crackprodukte. Im gleichen Zeitraum sank der Anfall an 3-Methylbutenal von 218 Teilen pro Stunde auf 211 Teile ab, entsprechend einem Ausbeuteabfall von 76,7% auf 74,2%, bezogen auf umgesetztes 3-Methyl-3-buten-1-ol.

## Patentansprüche

1. Verfahren zur kontinuierlichen Herstellung von 3-Alkyl-buten-1-alen der Formel

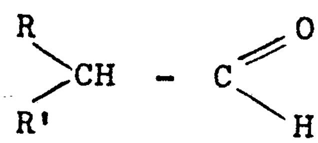 I

in der R ein Wasserstoffatom und R′ einen $H_2C=C(CH_3)$-Rest oder beide Reste R und R′ zusammen den Rest der Formel $H_3C-C(CH_3)=$ bedeuten, durch oxidierende Dehydrierung von 3-Alkyl-buten-1-olen der Formel

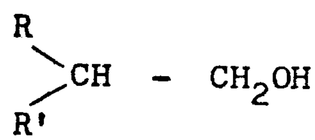 II

in der R und R′ die obengenannte Bedeutung haben, bei der man die 3-Alkyl-buten-1-ole bei Temperaturen von 320 bis 650° C mit Sauerstoff über einen Kupfer und/oder Silber enthaltenden Katalysator leitet, die Reaktionsgase abkühlt und die 3-Alkyl-buten-1-ale abtrennt, dadurch gekennzeichnet, dass man das dampfförmige Reaktionsgemisch, das eine Temperatur von 320 bis 650° C aufweist, innerhalb von einer Sekunde nach der Berührung mit dem Katalysator mit einer Flüssigkeit aus Wasser und/oder dem kondensierten Reaktionsgemisch der Temperatur von −20 bis 50° C in Berührung bringt und aus dem erhaltenen Kondensat die 3-Alkyl-buten-1-ale abtrennt.

2. Verfahren nach Anspruch 1, dadurch gekennzeichnet, dass man die Flüssigkeit aus Wasser und/oder dem kondensierten Reaktionsgemisch in Form von Tröpfchen mit einem durchschnittlichen Durchmesser von 1 bis 2000 Mikrometern mit den Reaktionsgasen in Berührung bringt.

3. Verfahren nach Anspruch 2, dadurch gekennzeichnet, dass die Hauptmenge der Tröpfchen auf den Strom der Reaktionsgase mit einem Winkel von 2 bis 85° zur Stromachse auftrifft.

## Claims

1. A process for the continuous preparation of 3-alkyl-buten-1-als of the formula

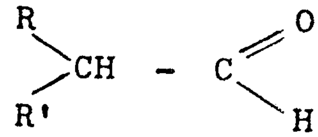 I

where R is hydrogen and R′ is $H_2C=C(CH_3)-$, or R and R′ together are $H_3C-C(CH_3)=$, by oxidative dehydrogenation of 3-alkyl-buten-1-ols of the formula

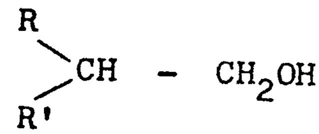 II

where R and R′ have the above meanings, by passing the 3-alkyl-buten-1-ols and oxygen over a catalyst containing copper and/or silver at from 320 to 650° C, condensing the reaction gases and separating off the 3-alkyl-buten-1-als, wherein, within one second after contact with the catalyst, the vaporous reaction mixture, which is at from 320 to 650° C, is brought into contact with a liquid consisting of water and/or the condensed reaction mixture at from −20 to 50° C, and the 3-alkyl-buten-1-als are separated off from the resulting condensate.

2. A process as claimed in claim 1, wherein the liquid consisting of water and/or the condensed reaction mixture is brought into contact with the reaction gases in the form of droplets having an average diameter of from 1 to 2,000 micrometers.

3. A process as claimed in claim 2, wherein most of the droplets impinge on the stream of reaction gases at an angle of from 2 to 85° to the axis of the stream.

## Revendications

1. Procédé pour la préparation en continu de 3-alkyl-butène-1-als de la formule

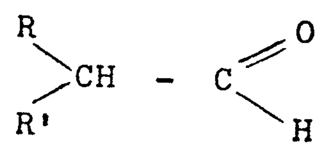 I

dans laquelle R désigne un atome d'hydrogène et R′ le groupe $H_2C=C(CH_3)-$ ou R et R′ forment ensemble un groupe de la formule $H_3C-C(CH_3)=$, par une déshydrogénation oxydante de 3-alkyl-butène-1-ols de la formule

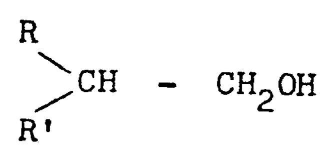 II

où R et R' possèdent la signification définie, dans laquelle on fait passer les 3-alkyl-butène-1-ols entre 320 et 650° C en présence d'oxygène sur un catalyseur au cuivre et(ou) à l'argent, on refroidit les produits réactionnels gazeux et on sépare les 3-alkyl-butène-1-als, caractérisé en ce que le mélange réactionnel gazeux, qui se trouve à une température entre 320 et 650° C, après le contact avec le catalyseur, est refroidi en l'espace d'une seconde par contact avec un liquide formé d'eau et (ou) de mélange réactionnel condensé, se trouvant entre −20 et 50° C, les alkyl-butène-1-als étant ensuite séparés du condensat obtenu.

2. Procédé suivant la revendication 1, caractérisé en ce que les gaz réactionnels sont mis en contact avec le liquide formé d'eau et(ou) de mélange réactionnel condensé sous forme de gouttelettes d'un diamètre moyen de 1 à 2000 µm.

3. Procédé suivant la revendication 2, caractérisé en ce que la majeure partie des gouttelettes rencontre le courant des gaz réactionnels sous un angle de 2 à 85° avec l'axe du courant.

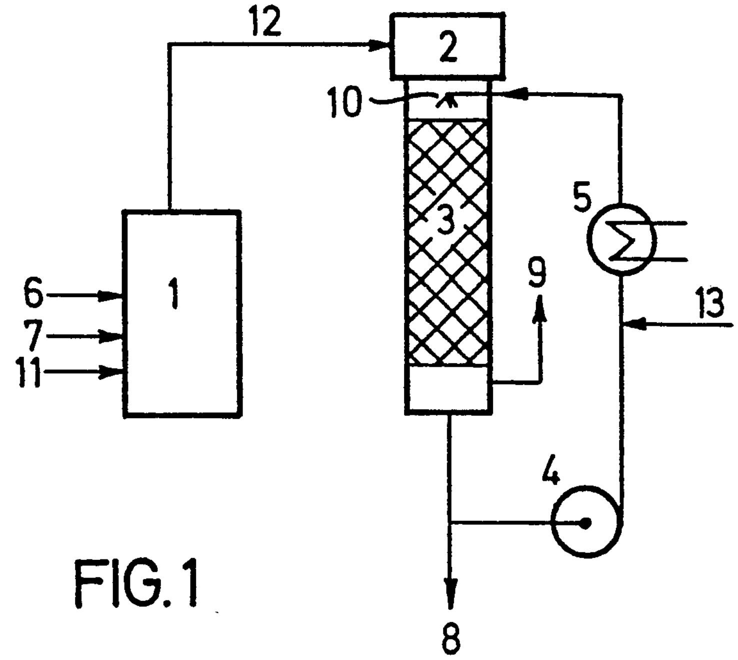


FIG. 1

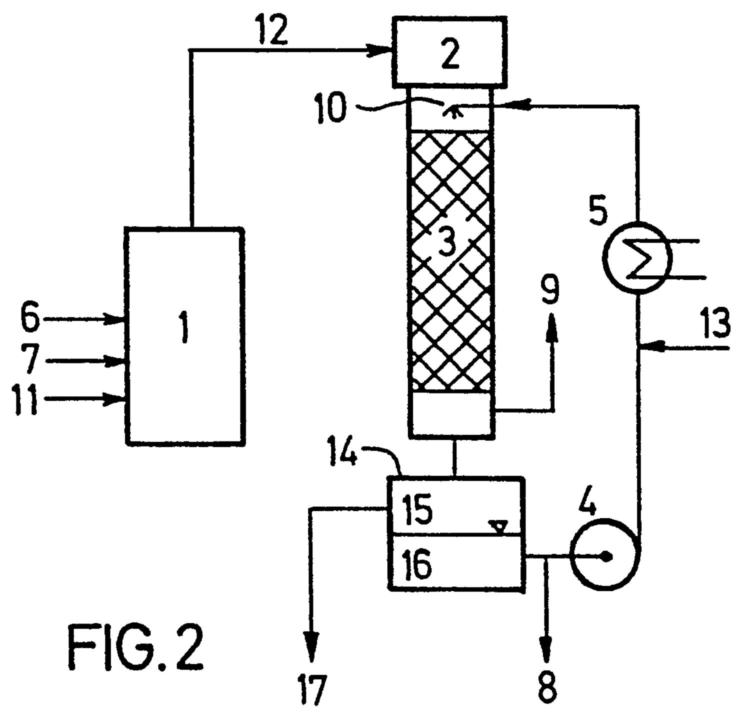


FIG. 2

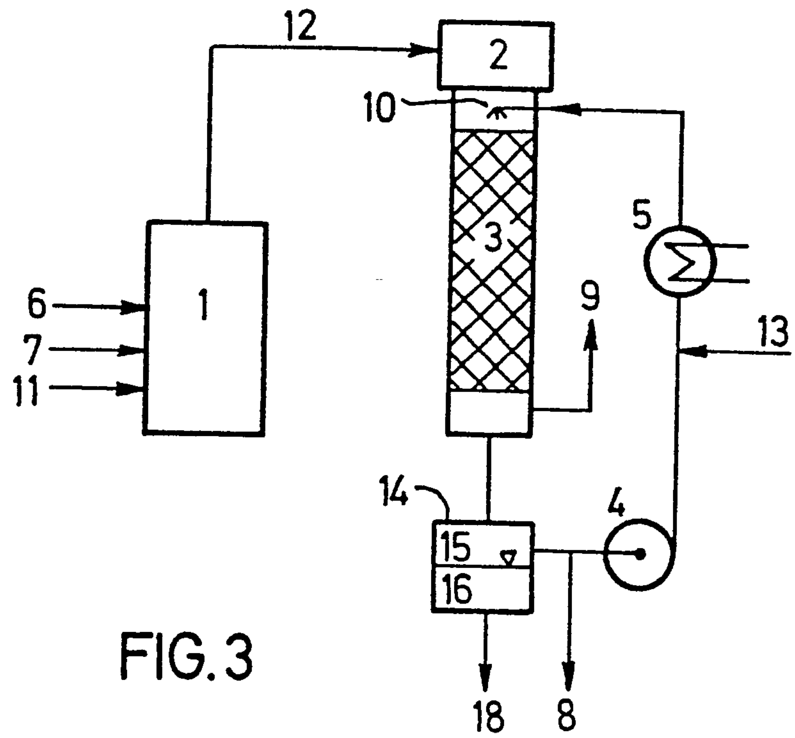
12
2
10
3
5
9
13
1
6
7
11
14
4
15
16
18
8

FIG.3

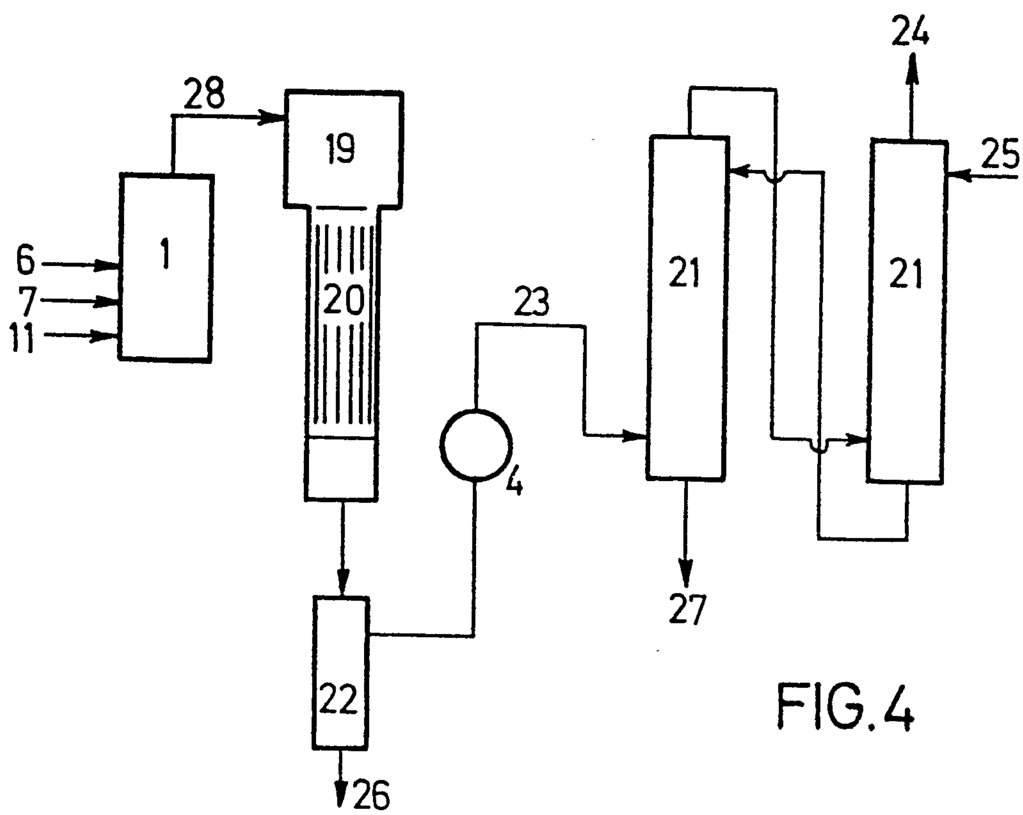
24
28
19
25
21
21
1
6
7
11
20
23
4
27
22
26

FIG.4